# EUROPEAN PATENT APPLICATION

(11) **EP 4 804 126 A1**
(43) Date of publication of application: **09.09.2026**
(21) Application number: 26162384.7
(22) Date of filing: 04.03.2026
(51) Int. Cl.: G06T 7/70, G06T 19/00

(54) **MEDICAL DEVICE AND OPERATION METHOD THEREOF**

(30) Priority: 04.03.2025 JP 2025033502
(71) Applicant: FUJIFILM Corporation, Tokyo 106-8620 (JP)
(72) Inventor: MACHII, Yusuke, Tokyo, 106-8620 (JP)
(74) Representative: Dehns Germany Partnerschaft mbB

(57) **Abstract**

The present disclosure provides a medical device and an operation method thereof that facilitate determination of which part of a 3D organ model not being observed corresponds to a region in an observation video.

Virtual field-of-view information in a Viewer coordinate system is estimated from an observation video. A first field-of-view 3D organ model is acquired by changing a 3D organ model into a first field-of-view display mode based on the virtual field-of-view information. The first field-of-view 3D organ model and the 3D organ model are displayed. The virtual field-of-view information is estimated by using any of a learning model that has been trained using the observation video and the virtual field-of-view information, coordinate information of a robot arm, or a field-of-view detection sensor.

## Description

### BACKGROUND OF THE INVENTION

### 1. Field of the Invention

The present disclosure relates to a medical device used during surgery such as laparoscopic surgery, and an operation method thereof.

### 2. Description of the Related Art

JP1997-270023A (JP-H9-270023A) (corresponding to US5883933A) discloses that, in addition to displaying a pseudo three-dimensional image, a plurality of tomographic images before and behind a viewpoint of the pseudo three-dimensional image and a rectangular field-of-view frame are displayed to easily understand a viewpoint position and a line-of-sight direction of a three-dimensional image of an interior of an object currently being displayed.

### SUMMARY OF THE INVENTION

In a case where an organ is resected in a laparoscopic surgery or the like, it is important to understand where to start cutting the organ and which blood vessel is visible during the resection in order to perform the surgery safely. Currently, a surgeon often makes determinations mentally by associating a structure in an intraoperative laparoscopic video with a structure in a preoperative 3D organ model. This makes it difficult to instantly determine which part of the preoperative 3D organ model corresponds to the region being observed with the laparoscope, and a heavy burden is placed on the surgeon. In JP1997-270023A (JP-H9-270023A), the viewpoint of the three-dimensional image of the interior of the object is designated in the setting, and is not a viewpoint of the laparoscope used during the surgery, such as in the laparoscopic video.

An object of the present disclosure is to provide a medical device and an operation method thereof that facilitate determination of which part of a preoperative 3D organ model corresponds to a region in an observation video.

According to the present disclosure, there is provided a medical device comprising: a processor, in which the processor acquires an observation video of an observation target including an observation target organ currently being observed, estimates virtual field-of-view information in the observation target from the observation video, acquires a 3D organ model corresponding to the observation target organ, acquires a first field-of-view 3D organ model by changing the 3D organ model into a first field-of-view display mode based on the virtual field-of-view information, displays the first field-of-view 3D organ model and the 3D organ model, and estimates the virtual field-of-view information by using any of a learning model that has been trained using the observation video and the virtual field-of-view information, coordinate information of a robot arm, or a field-of-view detection sensor.

It is preferable that the processor acquire a second field-of-view 3D organ model by changing the 3D organ model into a second field-of-view display mode different from the first field-of-view display mode based on the virtual field-of-view information, and display the second field-of-view 3D organ model instead of or in addition to the 3D organ model.

It is preferable that the processor estimate posture information representing a posture of the observation target organ currently being observed, from the virtual field-of-view information, acquire a posture 3D organ model by changing the 3D organ model in a posture display mode based on the posture information, and display the posture 3D organ model instead of or in addition to the 3D organ model.

It is preferable that the processor estimate posture information representing a posture of the observation target organ currently being observed, from the observation video, acquire a posture 3D organ model by changing the 3D organ model in a posture display mode based on the posture information, and display the posture 3D organ model instead of or in addition to the 3D organ model.

It is preferable that the processor determine whether or not dual display of the first field-of-view 3D organ model and the 3D organ model is required, based on either the virtual field-of-view information, or the virtual field-of-view information and the 3D organ model, and display the first field-of-view 3D organ model and the 3D organ model in a case where the dual display is required, and display only the first field-of-view 3D organ model in a case where the dual display is not required.

It is preferable that the processor determine whether or not dual display of the first field-of-view 3D organ model and the 3D organ model is required, based on the observation video, and display the first field-of-view 3D organ model and the 3D organ model in a case where the dual display is required, and display only the first field-of-view 3D organ model in a case where the dual display is not required.

It is preferable that the processor estimate switching information indicating to which of a plurality of pieces of the virtual field-of-view information switching is to be performed, or to which of a plurality of relative postures with respect to the observation target organ switching is to be performed, based on any of the virtual field-of-view information, the observation video, or the virtual field-of-view information and the 3D organ model, acquire a scene-specific 3D organ model by changing the 3D organ model in a scene-specific display mode based on the switching information, and display the scene-specific 3D organ model instead of or in addition to the 3D organ model.

It is preferable that the second field-of-view display mode be configured to either display a virtual viewpoint included in the virtual field-of-view information in the 3D organ model or highlight a virtual field-of-view region based on the virtual field-of-view information in the 3D organ model. It is preferable that the virtual field-of-view region be determined taking into consideration information on an angle of view of a camera that captures the observation video. It is preferable that the observation video be a laparoscopic video.

It is preferable that the virtual field-of-view information include a field of view of a camera that captures the observation video, or a virtual viewpoint defined with a surgical tool as a viewpoint and information regarding a virtual field of view based on the virtual viewpoint. It is preferable that the processor be capable of acquiring the first field-of-view 3D organ model by reflecting a user input in the virtual field-of-view information. It is preferable that the first field-of-view display mode be configured to enlarge and/or translate the 3D organ model in association with the virtual field-of-view information.

According to the present disclosure, there is provided an operation method of a medical device, the operation method comprising: a step of acquiring an observation video of an observation target including an observation target organ currently being observed; a step of estimating virtual field-of-view information in the observation target from the observation video; a step of acquiring a 3D organ model corresponding to the observation target organ; a step of acquiring a first field-of-view 3D organ model by changing the 3D organ model into a first field-of-view display mode based on the virtual field-of-view information; and a step of displaying the first field-of-view 3D organ model and the 3D organ model, in which the virtual field-of-view information is estimated by using any of a learning model that has been trained using the observation video and the virtual field-of-view information, coordinate information of a robot arm, or a field-of-view detection sensor.

According to the present disclosure, it is possible to facilitate determination of which part of a preoperative 3D organ model corresponds to a region in an observation video.

### BRIEF DESCRIPTION OF THE DRAWINGS

FIG. 1 is a schematic diagram of a medical system.
FIG. 2 is a block diagram showing functions of a medical image processing device.
FIG. 3 is an image diagram of a laparoscopic video.
FIG. 4 is a block diagram showing a function of a field-of-view estimation unit.
FIG. 5A is an explanatory diagram showing estimation of virtual field-of-view information from a laparoscopic video in a case different from that in FIG. 5B, and FIG. 5B is an explanatory diagram showing estimation of virtual field-of-view information from a laparoscopic video in a case different from that in FIG. 5A.
FIG. 6 is an image diagram of a laparoscopic video and a 3D organ model.
FIG. 7 is a block diagram showing functions of a first field-of-view 3D organ model acquisition unit and a display controller.
FIG. 8A is an explanatory diagram showing a first field-of-view 3D organ model acquired from virtual field-of-view information in a case different from that in FIG. 8B, and FIG. 8B is an explanatory diagram showing a first field-of-view 3D organ model acquired from virtual field-of-view information in a case different from that in FIG. 8A.
FIG. 9 is an image diagram of a laparoscopic video, a first field-of-view 3D organ model, and a 3D organ model.
FIG. 10 is a block diagram showing functions of a first field-of-view 3D organ model acquisition unit, a second field-of-view 3D organ model acquisition unit, and a display controller.
FIG. 11 is an image diagram of a laparoscopic video, a first field-of-view 3D organ model, and a second field-of-view 3D organ model.
FIG. 12 is an image diagram of a laparoscopic video, a first field-of-view 3D organ model, and a second field-of-view 3D organ model, in which a virtual field-of-view region is emphasized in a different way from that in FIG. 11.
FIG. 13A is a block diagram showing functions of a posture 3D organ model acquisition unit, a first field-of-view 3D organ model acquisition unit, and a display controller, in which input information is different from that in FIG. 13B, and FIG. 13B is a block diagram showing functions of a posture 3D organ model acquisition unit, a first field-of-view 3D organ model acquisition unit, and a display controller, in which input information is different from that in FIG. 13A.
FIG. 14 is an explanatory diagram showing a three-dimensional coordinate system consisting of an X-axis, a Y-axis, and a Z-axis.
FIG. 15A is an image diagram of a posture 3D organ model, a first field-of-view 3D organ model, and a laparoscopic video that are different from those in FIG. 15B, and FIG. 15B is an image diagram of a posture 3D organ model, a first field-of-view 3D organ model, and a laparoscopic video that are different from those in FIG. 15A.
FIG. 16A is a block diagram showing a function of a display determination unit in a case where dual display is required, and FIG. 16B is a block diagram showing a function of a display determination unit in a case where dual display is not required.
FIG. 17A is an image diagram showing a case where dual display is required, and FIG. 17B is an image diagram showing a case where dual display is not required.
FIG. 18A is a block diagram showing functions of a scene-specific 3D organ model acquisition unit, a first field-of-view 3D organ model acquisition unit, and a display controller, in which input information is different from that in FIG. 18B, and FIG. 18B is a block diagram showing functions of a scene-specific 3D organ model acquisition unit, a first field-of-view 3D organ model acquisition unit, and a display controller, in which input information is different from that in FIG. 18A.
FIG. 19A is an image diagram of a scene-specific 3D organ model, a first field-of-view 3D organ model, and a laparoscopic video in a case of a laparoscopic field of view, FIG. 19B is an image diagram of a scene-specific 3D organ model, a first field-of-view 3D organ model, and a laparoscopic video in a case of a probe field of view.
FIG. 20 is a flowchart showing a series of flows of the present disclosure.

### DESCRIPTION OF THE PREFERRED EMBODIMENTS

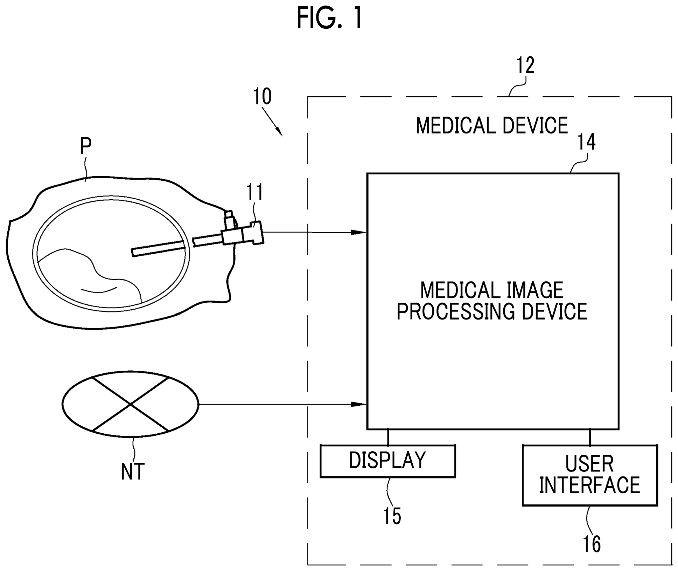

As shown in FIG. 1, a medical system 10 comprises a laparoscope 11 and a medical device 12. The laparoscope 11 captures an image of an inside of a body of a patient P and transmits a laparoscopic video obtained by the capturing to the medical device 12. The laparoscope 11 is also connected to a light source device (not shown), and illumination light from the light source device is supplied to the laparoscope 11.

The medical device 12 comprises a medical image processing device 14 configured by a computer such as a server, a display 15, and a user interface 16. In addition, the medical image processing device 14 is connected to a network NT. A picture archiving and communication system (PACS) or the like is connected to the network NT, and various image data and the like from the PACS are incorporated into the medical image processing device 14 via the network NT.

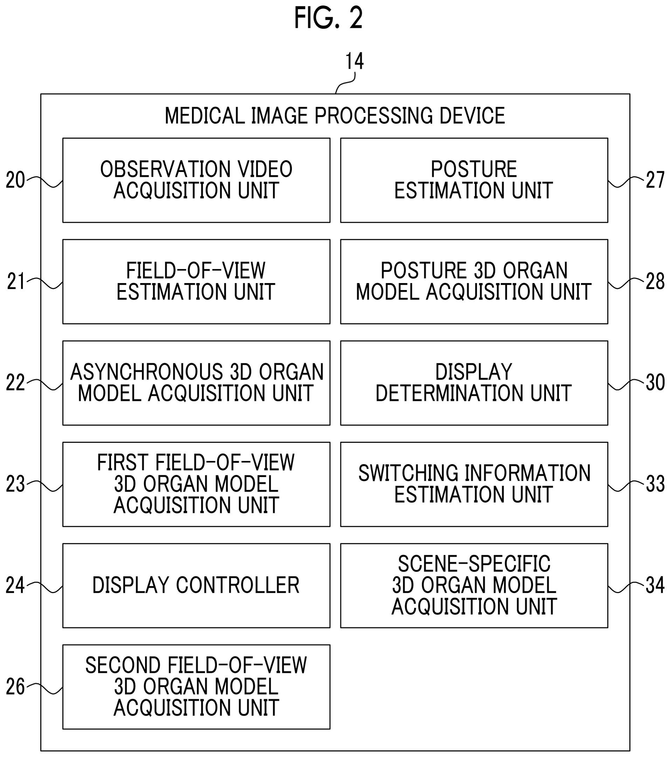

In the medical image processing device 14, a program for executing various types of processing is stored in a program memory (not shown). A central controller (not shown) configured by a processor executes the program in the program memory, whereby the medical image processing device 14 implements functions of an observation video acquisition unit 20, a field-of-view estimation unit 21, an asynchronous 3D organ model acquisition unit 22, a first field-of-view 3D organ model acquisition unit 23, a display controller 24, a second field-of-view 3D organ model acquisition unit 26, a posture estimation unit 27, a posture 3D organ model acquisition unit 28, a display determination unit 30, a switching information estimation unit 33, and a scene-specific 3D organ model acquisition unit 34, as shown in FIG. 2.

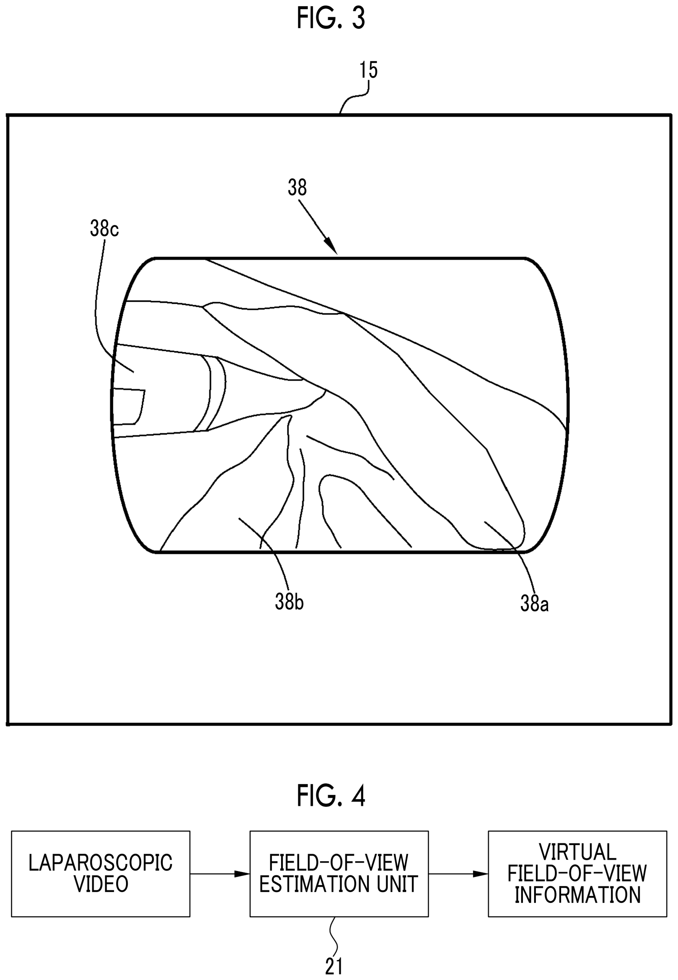

The observation video acquisition unit 20 acquires an observation video of an observation target including the observation target organ currently being observed. In the present embodiment, the laparoscopic video obtained by the laparoscope 11 is acquired as the observation video. The term "observation" refers to a period in which a user observes a video for observation such as the laparoscopic video, and includes not only a period in which the observation target organ is observed but also a period in which various surgeries such as resection of the observation target organ are performed during the observation. Specifically, as shown in FIG. 3, a laparoscopic video 38 includes a liver 38a, a structure 38b around the liver 38a, an ultrasound probe 38c that is one of various treatment tools, and the like. The observation target organ currently being observed corresponds to the liver 38a, and the observation target includes the liver 38a, as well as the structure 38b around the liver 38a and the ultrasound probe 38c. The laparoscopic video 38 is displayed on the display 15. The display of the laparoscopic video 38 is controlled by the display controller 24.

It is preferable that the observation video be a color video, and various medical videos other than the laparoscopic video may be used. In addition, in a case of the laparoscopic video, the video may be a monocular video captured by a single imaging sensor, or stereo videos captured by a plurality of imaging sensors. In addition, the observation video may be used in combination with the 3D organ model to estimate virtual field-of-view information.

As shown in FIG. 4, the field-of-view estimation unit 21 estimates virtual field-of-view information in an observation target (for example, a Viewer coordinate system) from the laparoscopic video. Specifically, during observation, the field of view of the laparoscope 11 changes depending on the situation, and thus the virtual field-of-view information in the observation target is estimated from the laparoscopic video 38, which is the observation video, in accordance with the change in the field of view. The virtual field-of-view information includes the field of view of the laparoscope 11, or a virtual viewpoint defined with a surgical tool as a viewpoint and information regarding a virtual field of view based on the virtual viewpoint. The surgical tool is, for example, the ultrasound probe 38c. The virtual field-of-view information can be represented by a three-dimensional coordinate system on the Viewer, as with posture information described below.

It is preferable that the field-of-view estimation unit 21 estimate the virtual field-of-view information by using any of a learning model that has been trained using the observation video and the virtual field-of-view information, coordinate information of a robot arm, or a field-of-view detection sensor.

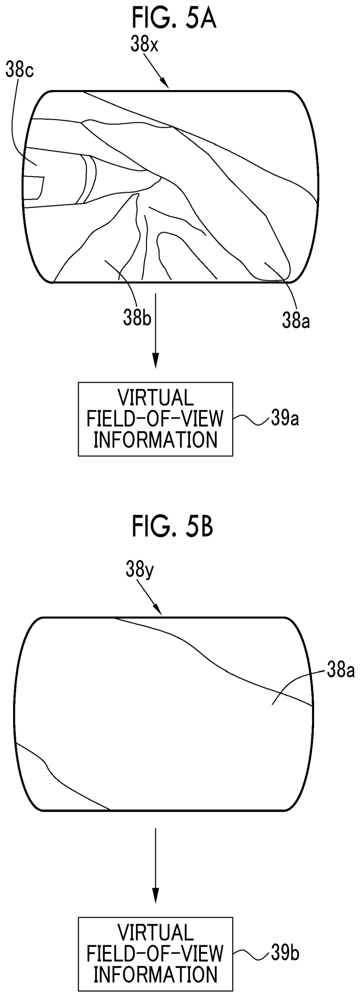

For example, as shown in FIG. 5A, in a case of a laparoscopic video 38x showing the ultrasound probe 38c on the left side and a liver 40a on the right side, virtual field-of-view information 39a including a virtual viewpoint and a virtual field of view based on the laparoscopic video 38x is estimated. On the other hand, in a case where the field of view of the laparoscope 11 changes, as shown in FIG. 5B, in a case of a laparoscopic video 38y in which a position of the liver 40a is moved to the center compared with the laparoscopic video 38x and only a part of the liver 40a is visible under magnified observation, virtual field-of-view information 39b including a virtual viewpoint and a virtual field of view based on the laparoscopic video 38y is estimated.

The asynchronous 3D organ model acquisition unit 22 acquires a 3D organ model corresponding to the observation target organ and asynchronous with the observation video. The 3D organ model that is asynchronous with the observation video is displayed together with the 3D organ model synchronized with the observation video based on the virtual field-of-view information. The 3D organ model is acquired from a 3D organ model image server (not shown) or the like via the network NT. The 3D organ model is a model extracted from a radiation image such as an X-ray image or a CT image, or an MRI image.

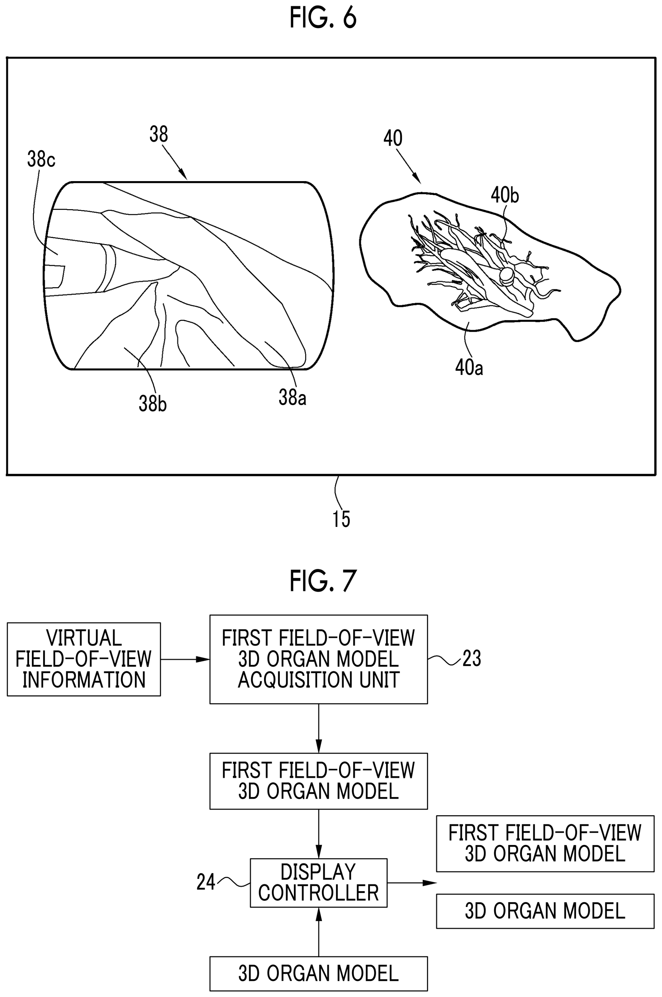

Specifically, as shown in FIG. 6, in a case where the observation target organ is the liver, a 3D organ model 40 showing the liver 40a and a blood vessel 40b inside the liver is displayed in parallel with the laparoscopic video 38 on the display 15. The display on the display 15 is controlled by the display controller 24. In addition to the liver, the observation target organ may be, for example, a kidney, a pancreas, a spleen, a uterus, a lung, a bronchus, an intracranial blood vessel, a prostate, or a nerve, and is not limited to the above organs and may be various other organs.

As shown in FIG. 7, the first field-of-view 3D organ model acquisition unit 23 acquires a first field-of-view 3D organ model by changing a 3D organ model into a first field-of-view display mode based on the virtual field-of-view information. In the present embodiment, changing the 3D organ model into the first field-of-view display mode means that a preoperative 3D organ model is displayed in association with a virtual field of view that is dynamically estimated according to a position, a size, or a range of the observation target organ on the laparoscopic video. For example, it is preferable that the first field-of-view display mode be configured to enlarge and/or translate the 3D organ model 40 in association with the virtual field-of-view information. It is preferable that the first field-of-view 3D organ model acquisition unit 23 be capable of acquiring the first field-of-view 3D organ model by reflecting a user input in the virtual field-of-view information. The user input is performed by using the user interface 16.

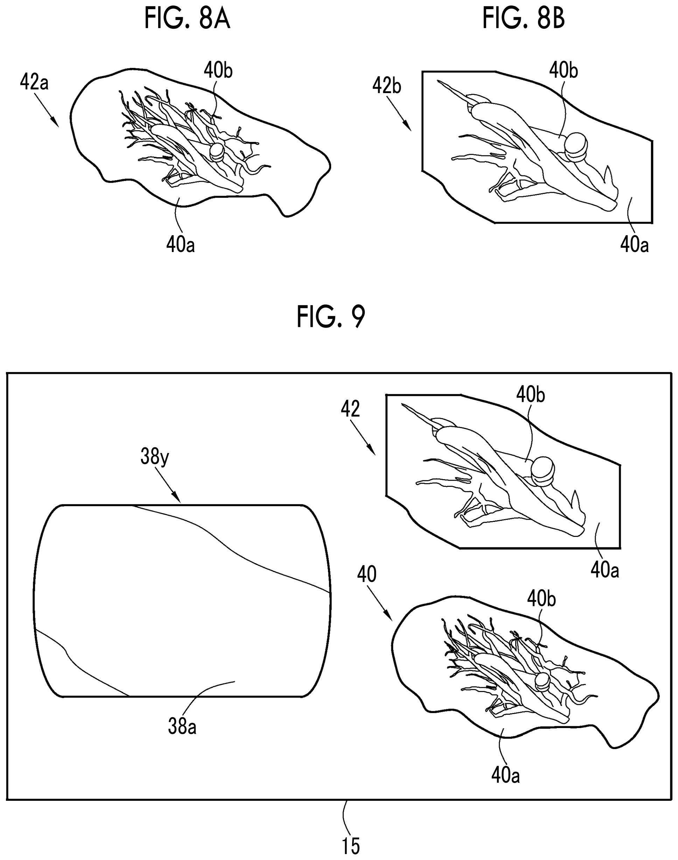

Specifically, in a case of the virtual field-of-view information 39a (see FIG. 5A) in which the overall image of the liver 38a is clearly visible in the laparoscopic video 38, as shown in FIG. 8A, a first field-of-view 3D organ model 42a that maintains the display mode of the 3D organ model 40 is acquired as the first field-of-view display mode. On the other hand, in a case of the virtual field-of-view information 39b (see FIG. 5B) in which only a part of the liver 38a is visible in the laparoscopic video 38 under magnified observation, as shown in FIG. 8B, a first field-of-view 3D organ model 42b that displays, from the display mode of the 3D organ model 40, only a portion corresponding to a region shown in the laparoscopic video 38 in accordance with the virtual field-of-view information 39b is acquired as the first field-of-view display mode.

The display controller 24 displays the first field-of-view 3D organ model and the 3D organ model (see FIG. 7). As a result, even in a case where the observation target organ is only partially displayed in the first field-of-view 3D organ model due to the magnified observation or the like, the overall image of the observation target organ can be checked by displaying the 3D organ model together. Specifically, as shown in FIG. 9, the first field-of-view 3D organ model 42b and the 3D organ model 40 are displayed on the display 15 in addition to the laparoscopic video 38. The first field-of-view 3D organ model 42b displays a part of the liver 40a that is displayed in the laparoscopic video 38 and its internal structure. On the other hand, the 3D organ model 40 displays the entire liver 40a and its internal structure.

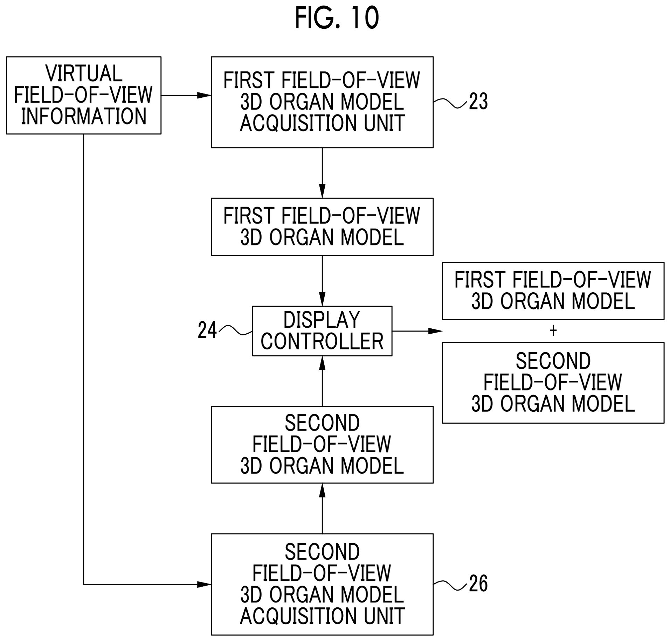

As shown in FIG. 10, the second field-of-view 3D organ model acquisition unit 26 acquires a second field-of-view 3D organ model by changing a 3D organ model into a second field-of-view display mode different from the first field-of-view display mode based on the virtual field-of-view information. In the present embodiment, changing the 3D organ model to the second field-of-view display mode means adding information on a position, a size, or a range of the observation target organ on the laparoscopic video that is changed in association with the virtual field of view and the virtual viewpoint. For example, as the second field-of-view display mode, it is preferable to display the virtual viewpoint in the 3D organ model 40 or highlight the virtual field-of-view region based on the virtual field of view in the 3D organ model 40. Then, the display controller 24 displays the second field-of-view 3D organ model instead of or in addition to the 3D organ model.

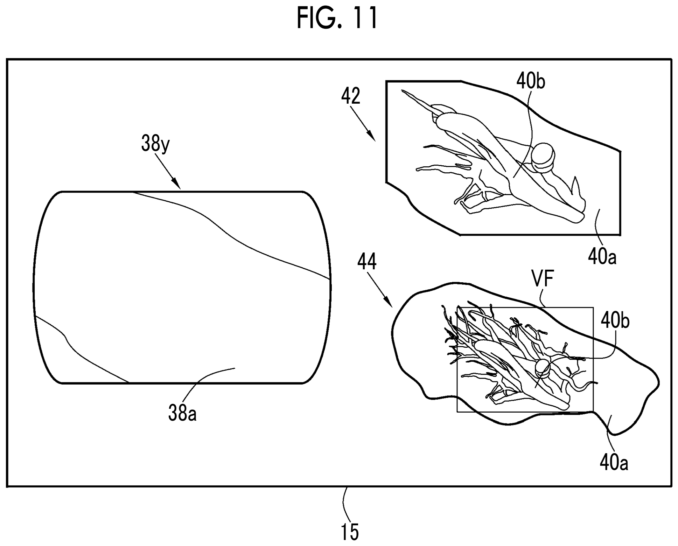

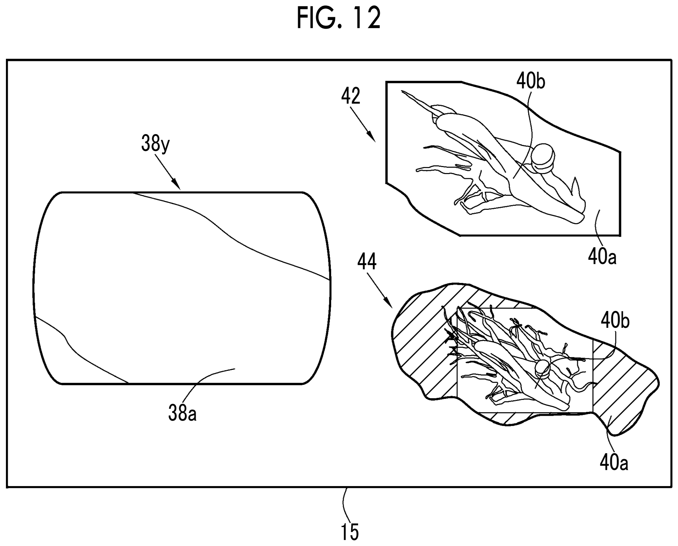

Specifically, as shown in FIG. 11, the laparoscopic video 38 and the first field-of-view 3D organ model 42b (see FIG. 9), and the second field-of-view 3D organ model 44 instead of the 3D organ model 40 are displayed on the display 15. The second field-of-view 3D organ model 44 highlights a virtual field-of-view region VF based on the position of the observation target organ on the laparoscopic video that is changed in association with the virtual field of view and the virtual viewpoint in the 3D organ model 40 by surrounding the virtual field-of-view region VF with a rectangle. As a result, it is possible to understand which part of the observation target organ being observed. As a method of highlighting the virtual field-of-view region VF, a region other than the virtual field-of-view region may be covered with a mask, as shown in FIG. 12. In addition, it is preferable that the virtual field-of-view region be determined taking into consideration on the angle of view of the laparoscope 11.

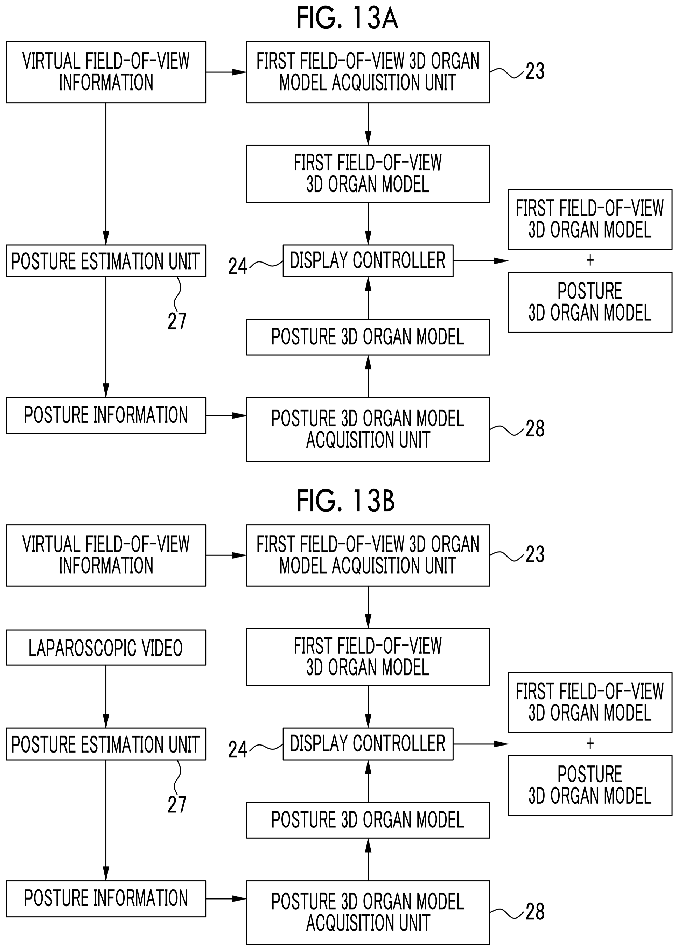

As shown in FIG. 13A, the posture estimation unit 27 estimates posture information representing the posture of the observation target organ currently being observed from the virtual field-of-view information. The posture 3D organ model acquisition unit 28 acquires a posture 3D organ model by changing the 3D organ model in a posture display mode based on the posture information. In the present embodiment, changing the 3D organ model in the posture display mode means changing the display mode in accordance with a change in the posture of the observation target organ caused by a direct action of the user or the like. The display controller 24 displays the posture 3D organ model instead of or in addition to the 3D organ model. As shown in FIG. 13B, the posture estimation unit 27 may estimate the posture information from the laparoscopic video instead of the virtual field-of-view information. The posture information may be represented by a relative posture between the laparoscope 11 and the observation target organ, or may be a relative posture between a surgical tool such as the ultrasound probe 38c and the observation target organ.

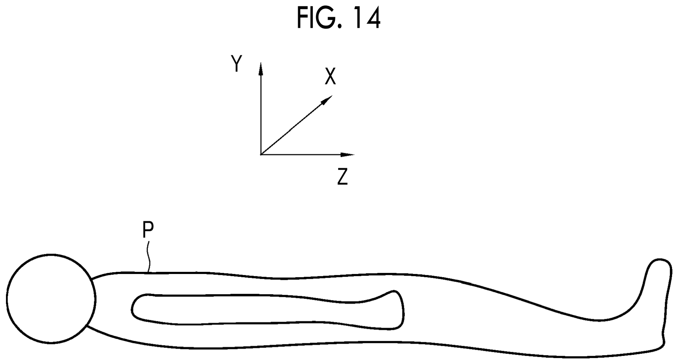

Specifically, the posture information represents the posture of the observation target organ in a three-dimensional coordinate system. As shown in FIG. 14, the three-dimensional coordinate system is represented by three axes of an X-axis, a Y-axis, and a Z-axis. The X-axis is represented by a positive value that is zero on a right side of the patient P and that increases toward a left side. The Y-axis is represented by a positive value that is zero on a ventral side of the patient P and that increases toward a dorsal side. The Z-axis is represented by a positive value that is zero on a head side of the patient P and that increases toward a foot side. The three-dimensional coordinate system may be a polar coordinate system represented by a radius and a polar angle in addition to an orthogonal coordinate system such as an X-axis, a Y-axis, and a Z-axis, and is not particularly limited.

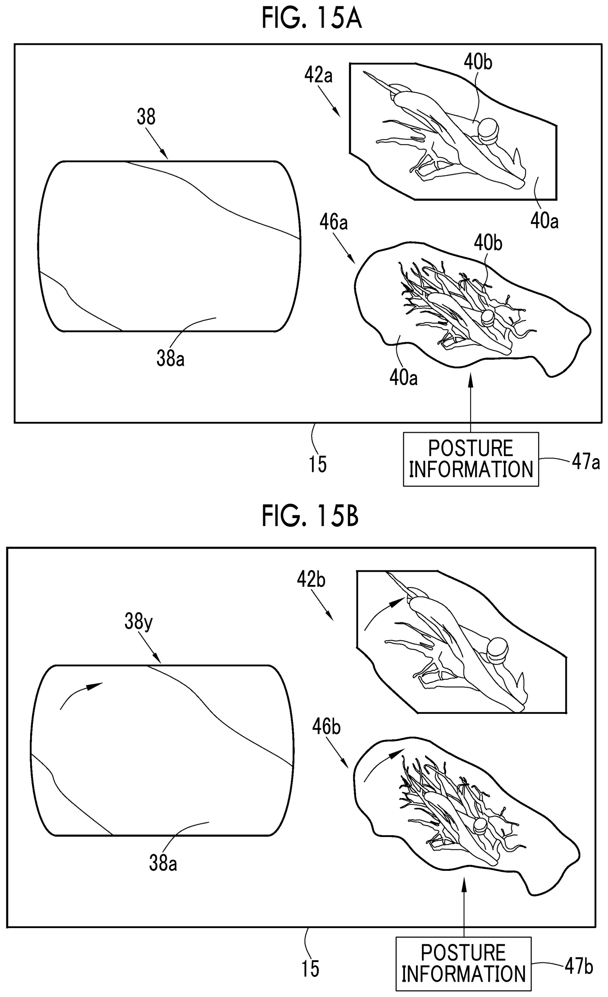

As shown in FIGS. 15A and 15B, the display controller 24 displays the laparoscopic video 38 and the first field-of-view 3D organ models 42a and 42b, and posture 3D organ models 46a and 46b instead of the 3D organ model 40 on the display 15. As shown in FIG. 15A, the posture 3D organ model 46a is a model acquired based on posture information 47a, and, as shown in FIG. 15B, the posture 3D organ model 46b is a model acquired based on posture information 47b different from the posture information 47a. It is preferable that the posture 3D organ model be a preoperative 3D organ model in which an internal structure such as a blood vessel is not enlarged, and be a model that is not in association with the virtual field-of-view information but is in association with only the posture of the observation target organ.

In the laparoscopic video 38, the liver 38a in FIG. 15B is rotated by a predetermined angle relative to the liver 38a in FIG. 15A due to the posture change of the liver 38a. In conjunction with this, the posture 3D organ model 46b is a model rotated relative to the posture 3D organ model 46a by a predetermined angle. In addition, the first field-of-view 3D organ model 42b is also a model rotated relative to the first field-of-view 3D organ model 42a by a predetermined angle.

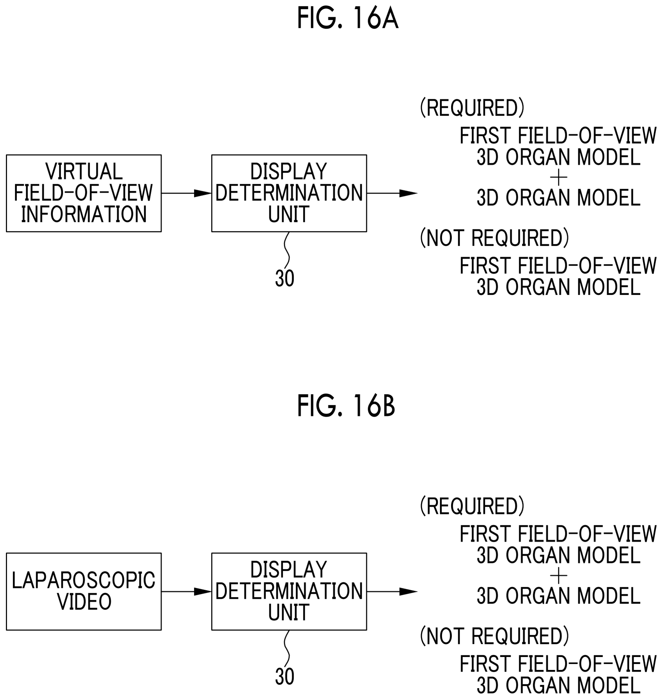

As shown in FIG. 16A, the display determination unit 30 determines whether or not dual display of the first field-of-view 3D organ model and the 3D organ model is required, based on the virtual field-of-view information. The display controller 24 displays the first field-of-view 3D organ model and the 3D organ model in a case where the dual display is required, and displays only the first field-of-view 3D organ model in a case where the dual display is not required. In this case, the display determination unit 30 may determine whether or not the dual display is required based on the asynchronous preoperative 3D organ model in addition to the virtual field-of-view information. As shown in FIG. 16B, the display determination unit 30 may determine whether the dual display is required based on the laparoscopic video. The user may be allowed to switch between the dual display and the single display (displaying only the first field-of-view 3D organ model).

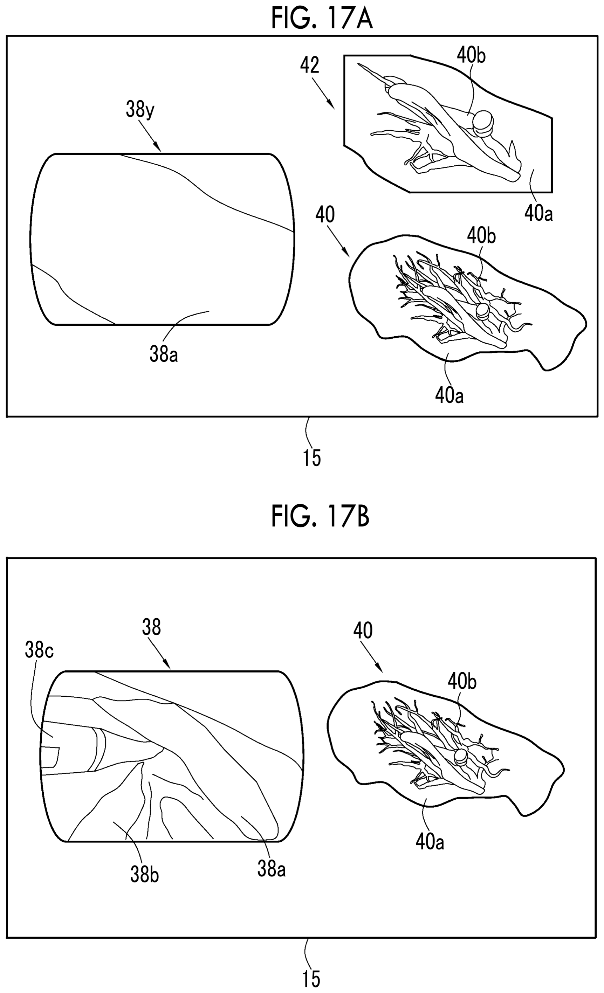

Specifically, in a case of the virtual field-of-view information 39b (see FIG. 5B) in which only a part of the liver 38a is visible in the laparoscopic video 38 under magnified observation, it is determined that the dual display is required, and, as shown in FIG. 17A, the first field-of-view 3D organ model 42b and the 3D organ model 40 are displayed on the display 15. On the other hand, in a case of the virtual field-of-view information 39a (see FIG. 5A) in which the overall image of the liver 38a is clearly visible in the laparoscopic video 38, it is determined that the dual display is not required, and, as shown in FIG. 17B, only the first field-of-view 3D organ model 42b is displayed on the display 15. In a case where the dual display is not required, the first field-of-view 3D organ model and the 3D organ model are similar to each other, so that there is no need for the dual display.

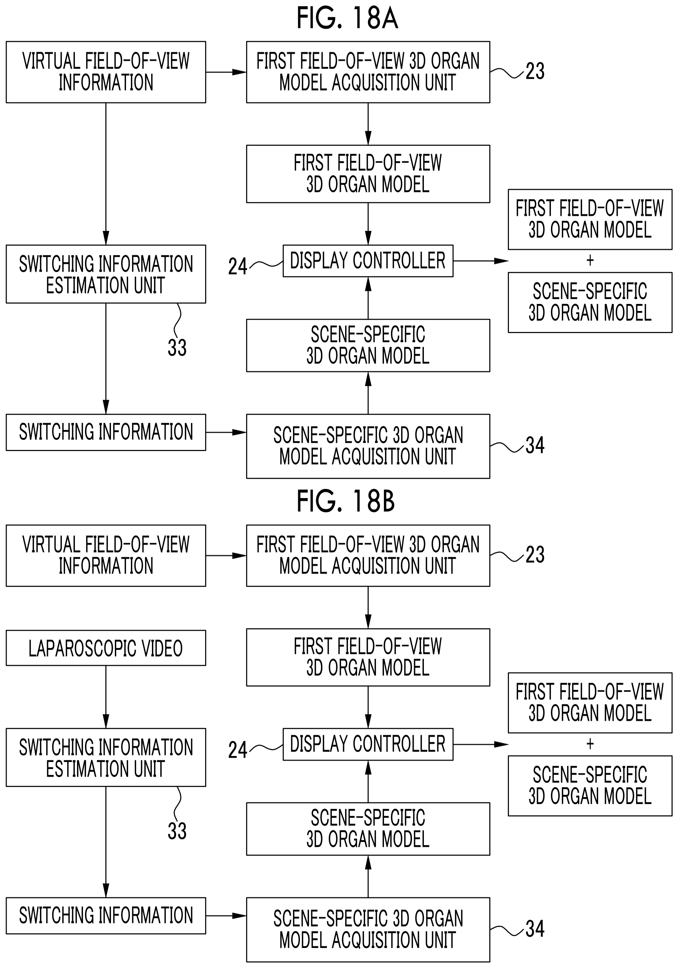

As shown in FIG. 18A, the switching information estimation unit 33 estimates switching information indicating to which of a plurality of pieces of the virtual field-of-view information switching is to be performed, or to which of a plurality of relative postures with respect to the observation target organ switching is to be performed, based on the virtual field-of-view information. Examples of the virtual field-of-view information to which switching is to be performed include a field of view of the laparoscope 11 and a virtual field of view defined with a surgical tool such as the ultrasound probe 38c as a viewpoint. In addition, examples of the relative posture to which switching is to be performed include a relative posture between the laparoscope 11 and the observation target organ and a relative posture between a surgical tool such as the ultrasound probe 38c and the observation target organ.

The scene-specific 3D organ model acquisition unit 34 acquires a scene-specific 3D organ model by changing the 3D organ model in a scene-specific display mode based on the switching information. In the present embodiment, changing the 3D organ model in the scene-specific display mode means changing the 3D organ model in accordance with a scene determined from the virtual field-of-view information or the relative posture to which switching is to be performed. Then, the display controller 24 displays the scene-specific 3D organ model instead of or in addition to the 3D organ model. As shown in FIG. 18B, the switching information estimation unit 33 may estimate the switching information based on the laparoscopic video. In addition, the switching information estimation unit 33 may estimate the switching information based on a combination of the virtual field-of-view information and the 3D organ model.

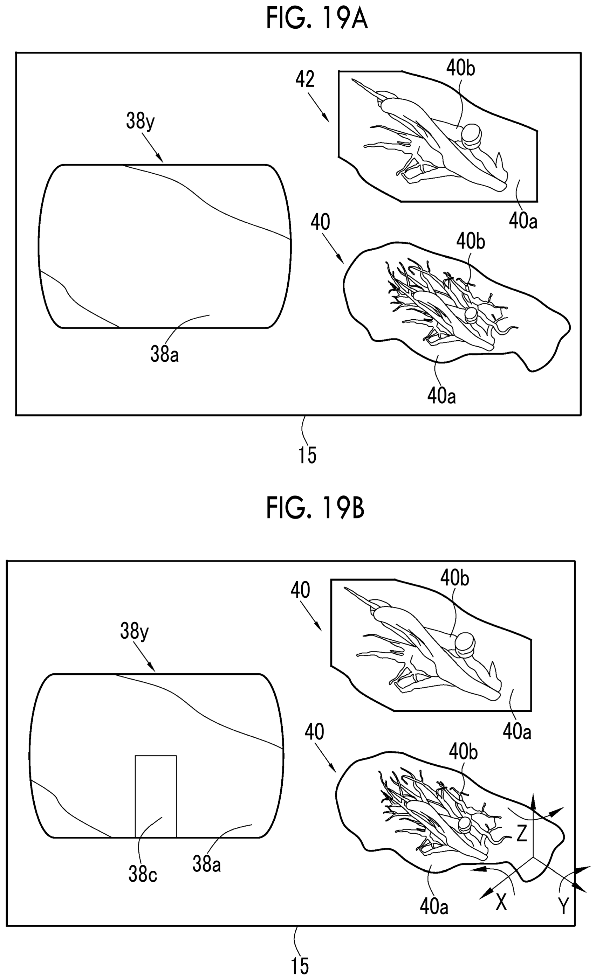

For example, as shown in FIG. 19A, in a case where only a part of the liver 38a is visible in the laparoscopic video 38 under magnified manner and no surgical tool is visible, it is estimated from the switching information that switching to the field of view of the laparoscope 11 is to be performed. In this case, a scene-specific 3D organ model 48a corresponding to the field of view of the laparoscope 11 is acquired. On the other hand, in a case where only a part of the liver 38a is visible in the laparoscopic video 38 under magnified manner and the ultrasound probe 38c as the surgical tool is visible, it is estimated from the switching information that switching to the probe virtual field of view defined with the ultrasound probe 38c as a viewpoint is to be performed. In this case, as shown in FIG. 19B, a scene-specific 3D organ model 48b is acquired by changing the 3D organ model in a scene-specific display mode according to the scene determined from the probe virtual field of view. The change based on the scene-specific display mode according to the scene is performed by rotating each of the three axes of the X-axis, the Y-axis, and the Z-axis of the three-dimensional coordinate system (in FIG. 19B, the rotation aspect is schematically shown by each axis).

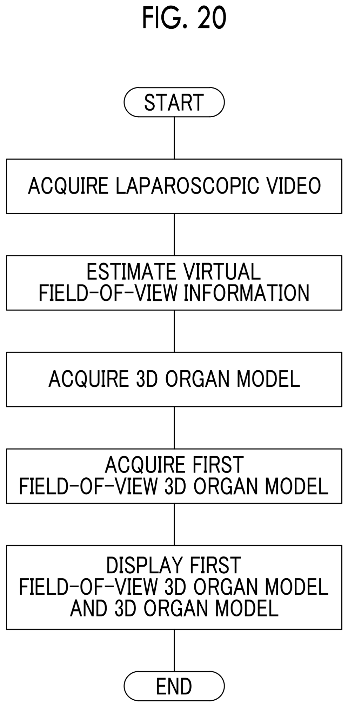

Next, a series of flows of the present disclosure will be described with reference to a flowchart of FIG. 20. The observation video acquisition unit 20 acquires the laparoscopic video from the laparoscope 11. The field-of-view estimation unit 21 estimates virtual field-of-view information in the observation target from the laparoscopic video. The asynchronous 3D organ model acquisition unit 22 acquires a 3D organ model corresponding to the observation target organ and asynchronous with the observation video. The first field-of-view 3D organ model acquisition unit 23 acquires a first field-of-view 3D organ model by changing a 3D organ model into a first field-of-view display mode based on the virtual field-of-view information. The display controller 24 displays the first field-of-view 3D organ model and the 3D organ model on the display 15. The above-described series of processing is repeatedly performed until the observation of the observation target is ended.

In the present embodiment, each process of the observation video acquisition unit 20, the field-of-view estimation unit 21, the asynchronous 3D organ model acquisition unit 22, the first field-of-view 3D organ model acquisition unit 23, the display controller 24, the second field-of-view 3D organ model acquisition unit 26, the posture estimation unit 27, the posture 3D organ model acquisition unit 28, the switching information estimation unit 33, and the scene-specific 3D organ model acquisition unit 34 is executed by any computer. In addition, any computer may execute the processing using a processor, a program, or a combination thereof. Any computer may be a general-purpose computer, a computer for a specific use, a system such as a workstation, or other hardware elements capable of executing a program. It is preferable that the posture estimation unit 27 and the switching information estimation unit 33 use a learning model as with the field-of-view estimation unit 21 and the like.

The processor may be configured by one or more pieces of hardware, and the type of hardware is not limited. For example, the processor may be configured by a programmable logic device such as a central processing unit (CPU), a micro processing unit (MPU), or a field programmable gate array (FPGA), a dedicated circuit for executing specific processing such as an application specific integrated circuit (ASIC), or hardware such as a graphics processing unit (GPU) or a neural processing unit (NPU). **In** addition, the processor has each unit or each means that executes various types of processing in the present embodiment. In addition, the types of hardware may be a combination of different types of hardware. In a case where a plurality of pieces of hardware are configured to execute one or a plurality of processes of a certain processor, the plurality of pieces of hardware may be present in devices physically separated from each other, or may be present in the same device. In addition, in any of the embodiments, the order of each processing executed by the processor is not limited to the above order and may be changed as appropriate. The hardware is configured by an electric circuit (circuitry) in which circuit elements such as semiconductor elements are combined.

Further, the present embodiment may be realized by hardware, software, firmware, microcode, or a combination thereof. Software, firmware, and microcode are configured by a program. In addition, the program may be, for example, a program module group, and each function thereof may be realized by a processor configured to execute each function. The program may be a program code or a plurality of code segments stored in one or a plurality of non-transitory computer-readable media (for example, a recording medium or other storage). The program may be divided and stored in a plurality of non-transitory computer-readable media present in devices physically separated from each other. The program code or the code segment may represent any combination of a procedure, a function, a subprogram, a routine, a subroutine, a module, a software package, a class, an instruction, a data structure, or a program statement. The program code or the code segment may be connected to another code segment or a hardware circuit by transmitting and receiving information, data, an argument, a parameter, or memory contents.

### Explanation of References

10: medical system
11: laparoscope
12: medical device
14: medical image processing device
15: display
16: user interface
20: observation video acquisition unit
21: field-of-view estimation unit
22: asynchronous 3D organ model acquisition unit
23: first field-of-view 3D organ model acquisition unit
24: display controller
26: second field-of-view 3D organ model acquisition unit
27: posture estimation unit
28: posture 3D organ model acquisition unit
30: display determination unit
33: switching information estimation unit
34: scene-specific 3D organ model acquisition unit
38, 38x, 38y: laparoscopic video
38a: liver
38b: structure
38c: ultrasound probe
39a, 39b: virtual field-of-view information
40: 3D organ model
40a: liver
40b: blood vessel
42, 42a, 42b: first field-of-view 3D organ model
44: second field-of-view 3D organ model
46a, 46b: posture 3D organ model
47a, 47b: posture information
48a, 48b: scene-specific 3D organ model
P: patient
NT: network
VF: virtual field-of-view region

## Claims

1. A medical device comprising:
a processor,
wherein the processor
acquires an observation video of an observation target including an observation target organ currently being observed,
estimates virtual field-of-view information of the observation target from the observation video,
acquires a 3D organ model corresponding to the observation target organ,
acquires a first field-of-view 3D organ model by changing the 3D organ model into a first field-of-view display mode based on the virtual field-of-view information,
displays the first field-of-view 3D organ model and the 3D organ model, and
estimates the virtual field-of-view information by using any of a learning model that has been trained using the observation video and the virtual field-of-view information, coordinate information of a robot arm, or a field-of-view detection sensor.

2. The medical device according to claim 1,
wherein the processor
acquires a second field-of-view 3D organ model by changing the 3D organ model into a second field-of-view display mode different from the first field-of-view display mode based on the virtual field-of-view information, and
displays the second field-of-view 3D organ model instead of or in addition to the 3D organ model.

3. The medical device according to claim 1 or 2,
wherein the processor
estimates posture information representing a posture of the observation target organ currently being observed, from the virtual field-of-view information,
acquires a posture 3D organ model by changing the 3D organ model into a posture display mode based on the posture information, and
displays the posture 3D organ model instead of or in addition to the 3D organ model.

4. The medical device according to any one of the preceding claims ,
wherein the processor
estimates posture information representing a posture of the observation target organ currently being observed, from the observation video,
acquires a posture 3D organ model by changing the 3D organ model into a posture display mode based on the posture information, and
displays the posture 3D organ model instead of or in addition to the 3D organ model.

5. The medical device according to any one of the preceding claims ,
wherein the processor
determines whether or not dual display of the first field-of-view 3D organ model and the 3D organ model is required, based on either the virtual field-of-view information, or the virtual field-of-view information and the 3D organ model, and
displays the first field-of-view 3D organ model and the 3D organ model in a case where the dual display is required, and displays the first field-of-view 3D organ model in a case where the dual display is not required.

6. The medical device according to any one of the preceding claims ,
wherein the processor
determines whether or not dual display of the first field-of-view 3D organ model and the 3D organ model is required, based on the observation video, and
displays the first field-of-view 3D organ model and the 3D organ model in a case where the dual display is required, and displays the first field-of-view 3D organ model in a case where the dual display is not required.

7. The medical device according to any one of the preceding claims ,
wherein the processor
estimates switching information indicating which of the plurality of pieces of virtual field-of-view information should be switched to, or which of a plurality of relative postures with respect to the observation target organ should be switched to, based on any of the virtual field-of-view information, the observation video, or the virtual field-of-view information and the 3D organ model,
acquires a scene-specific 3D organ model by changing the 3D organ model in a scene-specific display mode based on the switching information, and
displays the scene-specific 3D organ model instead of or in addition to the 3D organ model.

8. The medical device according to claim 2,
wherein the second field-of-view display mode is configured to either display a virtual viewpoint included in the virtual field-of-view information in the 3D organ model or highlight a virtual field-of-view region based on the virtual field-of-view information in the 3D organ model.

9. The medical device according to claim 8,
wherein the virtual field-of-view region is determined taking into consideration information on an angle of view of a camera that captures the observation video.

10. The medical device according to any one of claims 1 to 9,
wherein the observation video is a laparoscopic video.

11. The medical device according to any one of claims 1 to 10,
wherein the virtual field-of-view information includes a field of view of a camera that captures the observation video, or a virtual viewpoint defined using a surgical tool as a viewpoint, along with information regarding a virtual field-of-view based on the virtual viewpoint.

12. The medical device according to any one of claims 1 to 11,
wherein the processor is capable of acquiring the first field-of-view 3D organ model by reflecting a user input in the virtual field-of-view information.

13. The medical device according to any one of claims 1 to 12,
wherein the first field-of-view display mode is configured to enlarge and/or translate the 3D organ model in association with the virtual field-of-view information.

14. An operation method of a medical device, the operation method comprising:
a step of acquiring an observation video of an observation target including an observation target organ currently being observed;
a step of estimating virtual field-of-view information of the observation target from the observation video;
a step of acquiring a 3D organ model corresponding to the observation target organ;
a step of acquiring a first field-of-view 3D organ model by changing the 3D organ model into a first field-of-view display mode based on the virtual field-of-view information; and
a step of displaying the first field-of-view 3D organ model and the 3D organ model,
wherein the virtual field-of-view information is estimated by using any of a learning model that has been trained using the observation video and the virtual field-of-view information, coordinate information of a robot arm, or a field-of-view detection sensor.
